# EUROPEAN PATENT SPECIFICATION

(11) **EP 3 345 543 B1**
(45) Date of publication and mention of the grant of the patent: **15.03.2023**
(21) Application number: 16841761.6
(22) Date of filing: 27.08.2016
(51) Int. Cl.: A61B 5/026, A61B 5/02

(54) **BLOOD FLOW MEASUREMENT DEVICE**
BLUTFLUSSMESSVORRICHTUNG
DISPOSITIF DE MESURE DU DÉBIT SANGUIN

(30) Priority: 01.09.2015 JP 2015171602
(43) Date of publication of application: 11.07.2018
(73) Proprietor: Cosmotec Co., Ltd., Bunkyo Ward, Tokyo 113-0033 (JP); National University Corporation Tokyo Medical and Dental University, Bunkyo-ku Tokyo 113-8510 (JP)
(72) Inventor: INOUE Yoshinori, Tokyo 113-8510 (JP); KATSUI Sotaro, Tokyo 113-8510 (JP); OZAKI Takashi, Bunkyo Ward Tokyo 113-0033 (JP); YONEMARU Koji, Bunkyo Ward Tokyo 113-0033 (JP)
(74) Representative: Noréns Patentbyrå AB
(86) International application number: PCT/JP2016/075102
(87) International publication number: WO 2017/038729

(56) References cited:
- WO-A1-2010/131550
- WO-A1-2011/107771
- JP-A- 2004 529 703
- JP-A- 2008 113 876
- JP-A- 2012 005 595
- JP-A- 2013 013 547
- JP-A- 2014 518 730
- JP-A- 2015 047 246
- US-A1- 2010 241 025
- US-A1- 2015 112 206

## Description

### FIELD OF THE INVENTION

The present invention relates to a blood flow measurement device, and a blood flow measurement system.

### DESCRIPTION OF THE RELATED ART

A disease such as diabetes induces a blood circulatory disorder such as ischemia or the like of a lower extremity. For this reason, it is of quite importance to accurately evaluate the presence or absence of such blood circulatory disorder and the degree thereof in order to perceive a medical condition or the like. Thus, an evaluation of the blood circulation state has been performed using a blood flow measurement device that measures a blood flow rate of a biological body.

For example, the evaluation of the blood circulation state by use of the skin perfusion pressure (SPP) is performed by wrapping a cuff for pressurization around a lower extremity, measuring the blood flow rate while reducing the pressure from the state in which the blood vessel is avascularized by pressurizing, obtaining a cuff pressure (i.e., the SPP) when the blood flow is re-perfused, and evaluating the blood circulation state with the SPP being used as an index value.

As another example, the evaluation of the blood flow state by use of the transcutaneous partial pressure of oxygen (TcPO2) is performed by measuring the partial pressure of oxygen of a biological tissue while warming the biological tissue, and evaluating the blood circulation state with the partial pressure of oxygen being used as an index value.

Those conventional index values obtained by the above mentioned conventional methods have been appreciated in the medical community as appropriately evaluating the blood circulation state. In particular, those index values have been considered to be useful for evaluating the blood circulation state in a mass of tissue within a certain range including a muscle tissue or a thick blood vessel, such as an upper extremity or a lower extremity or the like, instead of the blood circulation in a mere skin surface.

### SUMMARY OF THE INVENTION

### Problems to be Solved by the Invention

However, when using the above mentioned conventional index values, in some cases, it has been difficult to obtain the index value, for example, the SPP imposes a heavy burden on a patient in the ischemic state as it is required to wrap a cuff around a lower extremity of the patient to pressurize the wrapped lower extremity. Therefore, it has been desired to pursue a new index value that is easily obtainable.

Also, the conventional index values are not necessarily capable of clearly distinguishing patient groups having different symptomatic states such as the blood circulatory disorder. For this reason, it has been desired to pursue a new index value that is capable of clearly distinguish a patient group having a bad blood circulation state from a patient group having a relatively good blood circulation state.

The present invention has been made in view of the above mentioned circumstances and object thereof is to obtain a new index value that is capable of clearly indicating a blood circulation state.

### Solution to the Problem

In order to solve the above mentioned problems, there is provided a method of evaluating a blood circulation state. The method comprises: a measuring step of measuring a blood flow rate of a biological tissue more than once over time; a slope calculating step of calculating a slope of a change over time in the blood flow rate measured in the measurement step; and an evaluating step for evaluating a blood circulation state in the biological tissue by comparing a value of the slope to a predetermined reference value.

According to the method of evaluating a blood circulation state of the present embodiments, a slope of the change over time in a blood flow rate is used as an index value for evaluating the blood circulation state. It is a newly found fact through a clinical trial performed by the inventors of the present invention that the slope serves as an index value that is capable of clearly indicating a blood circulation state.

Also, through the clinical trial, it has been confirmed that the value of the slope is capable of clearly indicating a blood circulation state within a mass of tissue including an underlying portion beneath a superficial layer (such as a muscle or the like), even if the measurement object of the blood flow rate is the superficial layer of a biological tissue (such as a surface skin).

Yet furthermore, through the clinical trial, it has been newly found out that, when using the slope as the index value, the distribution of the index values significantly differs between a subject group having a considerably deteriorated blood circulation state, such as the ischemia or the like, and a subject group having a blood circulation state that has not reached to the deteriorated extent.

The method of evaluating the blood circulation state according to the present disclosure has been conceived based on the above mentioned heuristic fact. According to the method of evaluating the blood circulation state it makes it possible to obtain a new index value that is capable of clearly indicating the blood circulation state so that it is achievable to evaluate the blood circulation state in an accurate manner based on such index value.

In order to solve the above mentioned problems, according to another aspect of the present disclosure there is provided a blood flow measurement device. The blood flow measurement device comprises: a measurement unit configured to measure a blood flow rate of a biological tissue more than once over time; a slope calculation unit configured to calculate a slope of a change over time in the blood flow rate measured by the measurement unit; and a display unit configured to display a value of the slope calculated by the slope calculation unit.

According to the blood flow measurement device of the present embodiments, it makes it possible to obtain a slope of the change over time in the blood flow rate so that the slope is capable of being used for evaluating a blood circulation state as an index value that is capable of clearly indicating the blood circulation state.

As mathematical methods for analyzing the amount that changes over time, various methods have been known such as, even limited to, for example, an analysis in connection with the decrease in the amount, a first order differentiation (slope or inclination), a second order differentiation, a decreasing rate (a ratio of the decline to an original amount), an attenuation rate (a ratio of an amount after attenuation to an original amount), a decay constant, a half-life period (half width at half maximum), a time constant or the like. This is why, it is never easy to find out an appropriate analytical method that can obtain an index value that is capable of clearly indicating the blood circulation state out of numerous analytical methods even considering the time and effort only for confirming whether the index value is appropriate or not through the clinical trial.

In particular, it is even more burdensome when trying to find out a particular index value that has the reliability comparable to the conventional SPP or TcPO2.

In addition, although a measurement object for measuring the blood flow rate is a superficial layer (for example, a skin) of a biological tissue, it is required for evaluating the blood circulation state to find out an index value that is capable of accurately evaluating the blood circulation state within a mass of tissue having a certain size (that is, a mass of tissue including an underlying portion beneath the superficial layer as well), such as an upper extremity, a lower extremity, a forearm (antebrachium) or a lower leg, or a buttock or the like. Requiring such analytical method for such index value differs in meaning from the analysis of the measured value itself.

On the other hand, from the perspective of medical technology, it is never useful to mount onto a blood flow measurement device the function to calculate a mathematical analytic value to display without confirming the medical reliability as the index value through the clinical trial or the like. To the contrary, it increases a certain "noise" so that the usefulness will be deteriorated.

The inventors of the present invention have newly found out the fact that, through the clinical trial performed this time, a slope of a change over time in the blood flow rate serves as an index value that is capable of clearly indicating the blood circulation state (in particular, the blood circulation state within the above mentioned mass of tissue). This kind of slope had been considered to be neither a common sense nor conventional as an index value of the blood circulation state before the inventors of the present invention confirmed the reliability thereof. For this reason, a method of evaluating a blood circulation state and a blood flow measurement device according to the present embodiments suffice to draw a line from the common sense or the conventional technique in the conventional medical technology.

According to the blood flow measurement device of the present embodiments, preferably, the above described measurement unit may be configured to irradiate the biological tissue with light, receive return light, and measure the blood flow rate based on a photo-detective signal received. According to this preferable blood flow measurement device, it makes it possible to measure the blood flow rate by a non-invasive process so as to reduce the burden on the biological tissue. Also, as the blood flow rate is measured from the blood flow itself by use of light, it makes it possible to accomplish the measurement with higher accuracy on the change particularly in the blood flow rate.

Yet furthermore, according to the blood flow measurement device of the present embodiments, more preferably, the above described measurement unit may be configured to transmit and receive light to and from the biological tissue by using a probe affixed to the biological tissue. According to this more preferable blood flow measurement device, it makes it possible to suppress the entire movement of the biological tissues, such as a body motion of a patient or the like, from affecting the measurement of the blood flow rate. As a result, it makes it possible to measure the blood flow rate with higher accuracy.

In order to solve the above mentioned problems, there is provided a blood flow measurement system. The blood flow measurement system comprises: a stimulus applying device configured to apply a stimulus that causes a blood flow rate to change with respect to a biological tissue; and a blood flow measurement device configured to measure the blood flow rate in the biological tissue while the stimulus applying device is applying the stimulus to the biological tissue or thereafter. The blood flow measurement device comprises: a measurement unit configured to measure a blood flow rate of the biological tissue more than once over time; a slope calculation unit configured to calculate a slope of a change over time in the blood flow rate measured by the measurement unit; and a display unit configured to display a value of the slope calculated by the slope calculation unit.

According to the blood flow measurement system of the present embodiments, it makes it possible to obtain a slope of the change over time in the blood flow rate by the blood flow measurement device. Thus, it makes it possible to use the slope of the change over time in the blood flow rate as an index value that is capable of clearly indicating the blood circulation state. In addition, as the stimulus applying device applies the stimulus to the biological tissue, it accelerates the change in the blood flow rate so as to obtain the index value easily.

Yet furthermore, according to the blood flow measurement system of the present embodiments, preferably, the stimulus applying device may be configured to warm the biological tissue; and the blood flow measurement device may be configured to measure the blood flow rate after the biological tissue being warmed.

According to this preferable blood flow measurement system, it makes it possible to obtain an appropriate index value while assuring the safety by using the warming, which is relatively less stimulating and puts less burden on the biological tissue, out of variously conceivable stimuli such as a warming, a cooling, a friction, an oscillation, a chemical stimulus or the like.

### ADVANTAGEOUS EFFECT OF THE INVENTION

According to the present invention, it makes it possible to obtain an index value that is capable of clearly indicating the blood circulation state.

### BRIEF DESCRIPTION OF THE DRAWINGS

Fig. 1 is an appearance view schematically illustrating an exemplary blood flow measurement device according to a present embodiment of the present invention;
Fig. 2 is a functional block diagram illustrating an exemplary functional configuration of the blood flow measurement device;
Fig. 3 is a view illustrating an exemplary display screen by a GUI unit;
Fig. 4 is a view illustrating a measurement example of the blood flow rate of a healthy subject;
Fig. 5 is a view illustrating a measurement example of the blood flow rate of a patient with ischemia in a lower extremity;
Fig. 6 is a graph exemplarily illustrating a correlativity between the slope of the blood flow rate and TcPO2; and
Fig. 7 is a graph exemplarily illustrating a separation action obtainable when using the slope of the blood flow rate as the index value.

### DETAILED DESCRIPTION OF THE INVENTION

Hereinafter, embodiments of the present invention will be explained in detail with reference to accompanying drawings.

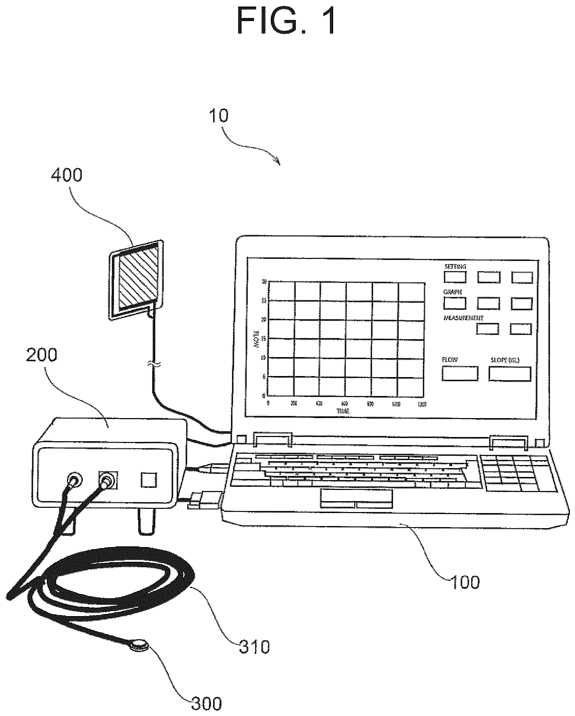

Fig. 1 is an appearance view illustrating a certain embodiment of a blood flow measurement system of the present invention.

The blood flow measurement system 10 according to the present embodiment has a configuration in which a light emitting and light receiving unit 200 that emits and receives laser light and a warming sheet 400 are connected to a personal computer 100, respectively.

The warming sheet 400 is to be affixed to a biological tissue (for example, an arm or a leg or the like of a subject) and heats the biological tissue.

The laser light emitted from the light emitting and light receiving unit 200 is guided to a probe 300 through an optical fiber 310.

The probe 300 is also to be affixed to the biological tissue (for example, an arm or a leg or the like of a subject) and irradiates the biological tissue with the light. Also, the probe 300 receives return light returned from the biological tissue and transmits the return light to the optical fiber 310.

The light transmitted from the probe 300 through the optical fiber 310 is received by the light emitting and light receiving unit 200 and then transformed into an electric photo-detective signal that represents a light intensity of reception. The photo-detective signal is transmitted from the light emitting and light receiving unit 200 to the personal computer 100.

Next, a function of the above mentioned blood flow measurement system 10 will be described.

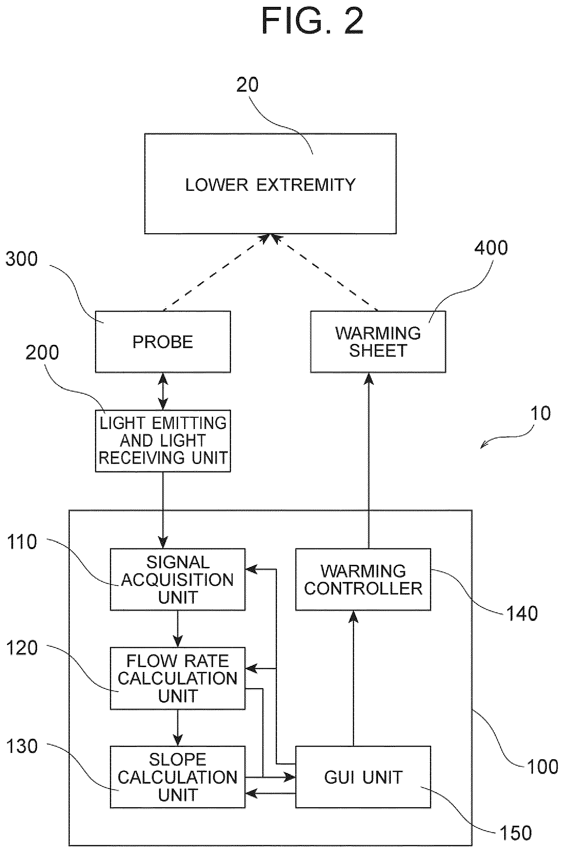

Fig. 2 is a functional block diagram illustrating a functional configuration of the blood flow measurement device.

As described above, the warming sheet 400 of the blood flow measurement system 10 is affixed to, for example, a lower extremity 20 of a subject and warms a portion thereof to which the warm sheet is affixed.

On the other hand, the probe 300 is affixed to the portion warmed by the warming sheet 400 in exchange for the warming sheet 400. It should be noted that, as the warming sheet 400 is a transparent sheet, it is possible to further affix the probe 300 onto the warming sheet 400 so as to perform the measurement while being warmed.

The personal computer 100 is provided with, as functional components, a signal acquisition unit 110, a flow rate calculation unit 120, a slope calculation unit 130, a warming controller 140, and a GUI unit 150. The warming sheet 400 is supplied with power from the personal computer 100 to perform warming, and the temperature thereof or the like is controlled by the warming controller 140.

The combination of the warming sheet 400 and the warming controller 140 corresponds to an illustrative embodiment of a stimulus applying device according to the present invention. Also, out of the blood flow measurement system 10, remaining portions except for the warming sheet 400 and the warming controller 140 corresponds to an illustrative embodiment of a blood flow measurement device.

The light emitted from the probe 300, which is affixed to the lower extremity 20 or the like of the subject and then returned therefrom, is received by the light emitting and light receiving unit 200 and then transformed into the photo-detective signal, as described above.

As the probe 300 is affixed to the biological tissue, it is possible to suppress the motion other than the blood flow, such as a body motion, from affecting the measurement. Also, the light emitted from the probe 300 and then scattered within the biological tissue contains a high proportion of the scattering component derived from the blood flow itself. By calculating the blood flow rate from the scattering component, it is possible to measure the blood flow rate in a direct and non-invasive manner. It should be noted that the blood flow rate measured in this phase is a blood flow rate in a capillary vessel of a skin of a subject. On the other hand, a blood flow rate in an underlying muscle or a thick blood vessel beneath the skin is not directly measured.

The photo-detective signals, which are obtained by the light emitting and light receiving unit 200, are then acquired over time by the signal acquisition unit 110 of the personal computer 100 and transmitted to the flow rate calculation unit 120, respectively.

The flow rate calculation unit 120 applies the Fast Fourier Transformation (FFT) to the photo-detective signals and calculates the frequency components thereof. The flow rate calculation unit 120 then calculates (i.e., measures) the blood flow rates from the signal intensity in the frequency range corresponding to the flow rate range of the blood flow multiple times, respectively. The calculation of the blood flow rate is continuously performed in parallel to the acquisition of the photo-detective signal over time by the signal acquisition unit 110. As a result, the blood flow rates for respective times are calculated over time.

A portion reaching to the flow rate calculation unit 120 from the probe 300 corresponds to an illustrative embodiment of the measurement unit according to the present invention.

The slope calculation unit 130 calculates the slope of change over time (temporal change) of the blood flow rate from the blood flow rate calculated by the flow rate calculation unit 120. The slope calculation unit 130 corresponds to an illustrative embodiment of the slope calculation unit according to the present invention. The time interval (calculation interval) may be manually set by a user, or alternatively set automatically. It is preferable in any event that the above time interval is a time interval that is after the transient increase, which will be described later.

The calculation result by the flow rate calculation unit 120 and the slope calculation unit 130 is displayed by the GUI unit 150 on a display device of the personal computer 100. Also, upon manipulation by a user through the GUI unit 150, various settings are instructed to the signal acquisition unit 110, the flow rate calculation unit 120, the slope calculation unit 130, and the warming controller 140. The GUI unit 150 corresponds to an illustrative embodiment of the display unit according to the present invention.

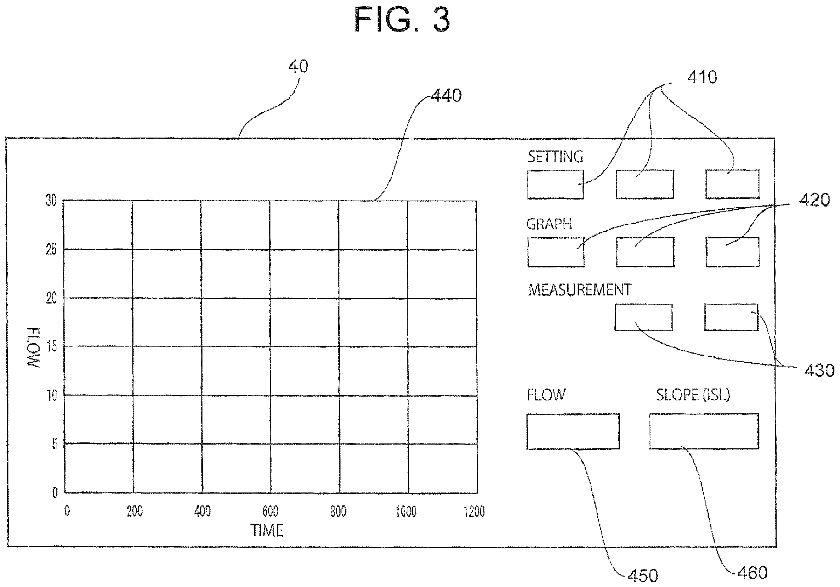

Fig. 3 is a view illustrating an exemplary display screen by the GUI unit.

The display screen 40 displayed by the GUI unit 150 on the display device is provided with functional buttons and display fields. By selecting the functional buttons through a pointing device or the like, it is possible to instruct various functions to the personal computer 100, and various calculation results are displayed on the display fields.

Likewise, by selecting each functional button 410 of the setting unit by a user, a display screen for each setting opens. Through the opened display screen for a particular setting, setting is made on a measurement condition such as a sampling frequency or measuring time or the like, a display condition of a measurement result, a calculation condition such as a calculation interval or the like of the slope, and a temperature condition for warming or the like. The measurement condition is set to the signal acquisition unit 110 in Fig. 2, the display condition is set to the GUI unit 150 itself, the calculation condition is set to the flow rate calculation unit 120 or the slope calculation unit 130 in Fig. 2, and the temperature condition is set to the warming controller 140 in Fig. 2, respectively.

Yet likewise, by selecting each functional button 430 of the measurement unit by a user, an instruction is made to start or stop of the measurement. A current value of the measured blood flow rate is displayed on the measurement value display field 450.

A graph display field 440 displays a graph that shows the blood flow rates over time, which are measured over time by the flow rate calculation unit 120.

Yet likewise, by selecting each functional button 420 of the graph manipulation unit by a user, it is instructed to save, read or print the displayed graph.

The slope display field 460 displays a value of the slope, which is calculated by the slope calculation unit 130 from the measured value of the blood flow rate, as the degree of the ischemic severity of the extremity (lower extremity).

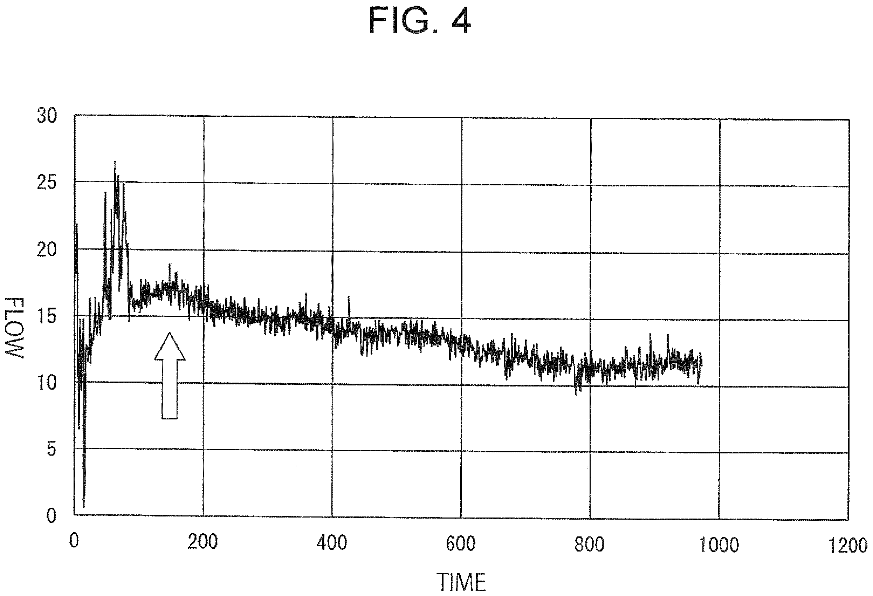

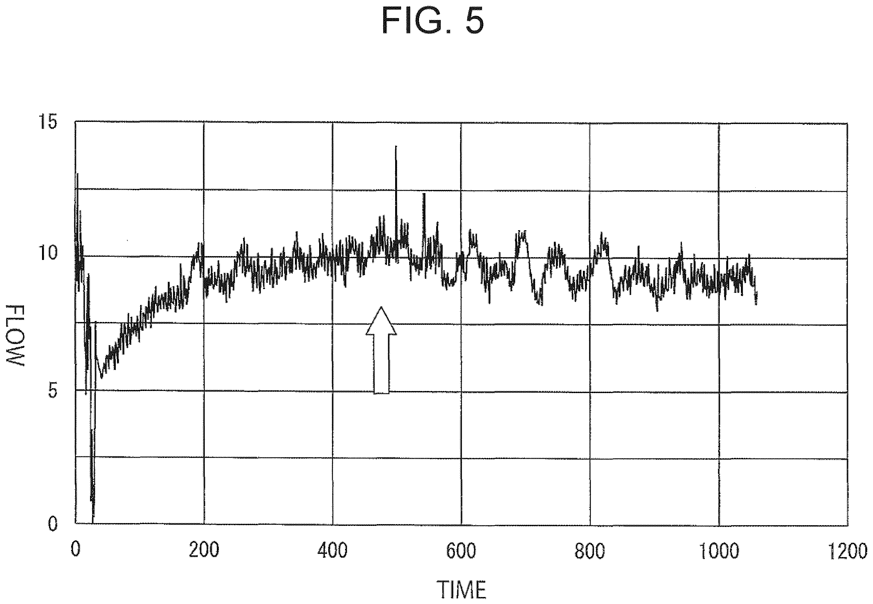

Fig. 4 is a graph illustrating an exemplary measurement result of the blood flow rate in a healthy subject, while Fig. 5 is a graph illustrating an exemplary measurement result of the blood flow rate in a patient with the ischemia in the lower extremity.

In Figs. 4 and 5, the horizontal axis denotes an elapsed time (second) and the vertical axis denotes the blood flow rate.

In the case of the healthy subject, as shown in Fig. 4, the blood flow rate increases for a while after stopping the warming, and then the blood flow rate gradually decreases. On the other hand, in the case of the patient with the ischemia in the lower extremity, as shown in Fig. 5, the blood flow rate less decreases, and in some cases, the blood flow rate hardly decreases.

Both in the case of the healthy subject and the case of the patient with the ischemia in the lower extremity, as shown by arrows in the figures, a temporal increase in the blood flow rate (hereinafter referred to as "transient increase") after stopping the warming is observed. However, a change in the blood flow rate after the transient increase significantly differs between the healthy subject and the patient with the ischemia in the lower extremity.

It is considered that the decrease in the blood flow rate after the warming (that is, the decrease after the transient increase) occurs by a mechanism in which the blood flow, which has been enhanced by warming, is reallocated from the warmed portion therearound. For this reason, when the blood circulatory state is deteriorated so as to cause the ischemic state, the blood flow is inhibited from being reallocated, and as shown in Fig. 5, a certain tendency is observed in which the decrease in the blood flow rate slows down.

The inventors of the present invention gave thought to a possibility that the slope of the blood flow rate might be appropriate as an index value of the blood circulatory state, and then verified an accuracy (i.e., reliability) of the slope of the blood flow rate as the index value through a clinical trial. It should be noted that, although values of a slope in a change that is temporally decreasing as shown in Figs. 4 and 5 are expressed as negative mathematically, it is assumed that a slope in a direction in which the blood flow rate is decreasing is expressed as positive according to the present embodiment.

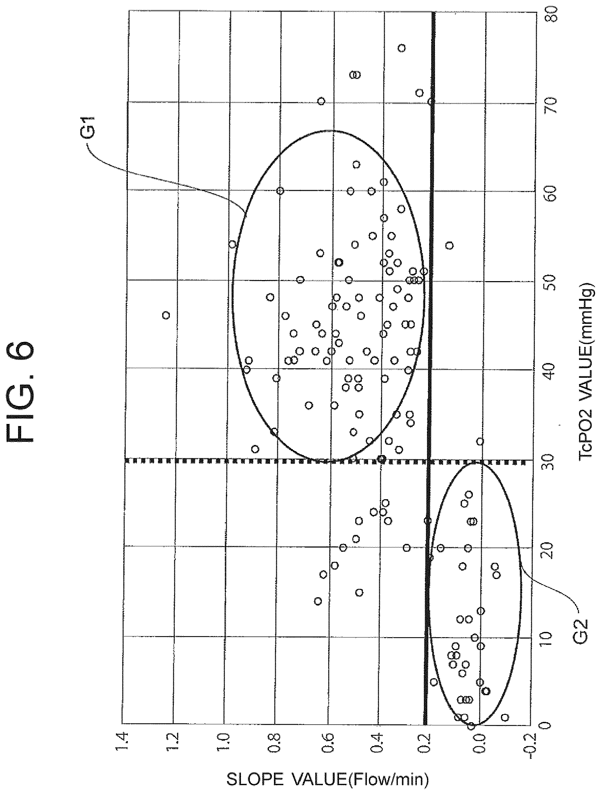

Fig. 6 is a graph illustrating the correlativity between the slope of the blood flow rate and the TcPO2.

In Fig. 6, the horizontal axis denotes a value of TcPO2 (mmHg) and the vertical axis denotes a slope of the blood flow rate (Flow/min).

As shown in Fig. 6, the graph plots the results in which the TcPO2 and the slopes of the blood flow rates are measured for numerous subjects.

The distribution of the plotted points on the graph is concentrated mainly on a first cluster G1 and a second cluster G2. The first cluster G1 is a group of subjects each having a slope of the blood flow rate equal to or greater than 0.20 and the TcPO2 value equal to or greater than 30 mmHg. The second cluster G2 is a group of subjects each having a slope of the blood flow rate less than 0.20 and the TcPO2 less than 30 mmHg.

The TcPO2 value less than 30 mmHg has been conventionally used in the medical community as a diagnostic criteria to make a diagnosis that the blood flow state in the lower extremity is a morbidity or a severe ischemia in the lower extremity. By applying this diagnostic criteria, the second cluster G2 is to be diagnosed as a morbidity or a severe ischemia in the lower extremity, while the first cluster G1 is to be diagnosed as having a relatively good blood circulatory state.

The fact that the plotted points are concentrated mainly in the first group G1 and the second group G2 suggests that a threshold value of "the slope of the blood flow rate of 0.20" substantially corresponds to the diagnostic criteria of "the TcPO2 value of 30 mmHg". For this reason, it is assumed that the slope of the blood flow rate has the reliability equivalent to the TcPO2 value as the index value indicating the blood circulatory state.

By performing the above described trial, it was confirmed that the slope of the blood flow rate serves as the index value having the reliability equivalent to the TcPO2. It should be noted that, although a diagrammatical illustration or the like is omitted, the inventors of the present invention have also verified the correlativity between the SPP and the slope of the blood flow rate. Through this verification, it was also confirmed that the slope of the blood flow rate serves as the index value having the reliability equivalent to the SPP.

In the meantime, as described above, while the measurement of the blood flow rate measures the blood flow rate in the capillary vessel of the skin, the blood circulation state evaluated by the index of the TcPO2 or SPP is a blood circulation state in a mass of tissue including the underlying muscles or the like beneath the skin. In other words, it is assumed that the quantity of state representing the blood circulation state of the mass of tissue is latent in the value of the blood flow rate of the skin. For this reason, it can be assumed that the quantity of state becomes actualized by using the slope of the blood flow rate.

More particularly, it can be considered that the slope of the blood flow rate of the skin represents the degree of the above described reallocation of the blood flow. Also, it can be considered that this reallocation of the blood flow occurs as a result that the blood vessel vasodilates and vasoconstricts in response to the stimulus in a vascular plexus residing in the mass of tissue including the portion beneath the skin as well.

In terms of those vasodilation and vasoconstriction of the blood vessel, it has been known that the blood vessel becomes scantily responsive when the ischemic state or the like occurs. Through the clinical trial this time, a certain result has been obtained that the second cluster G2, which has a bad blood circulation state, is proved to indicate the slope of change over time in the blood flow rate that is significantly smaller than that of the first cluster G1, which has a relatively good blood circulation state. It can be considered that this is because the above described exiguity in the responsiveness within the mass of tissue becomes actualized by way of the index value of "slope of the blood flow rate".

For this reason, it is assumed that the slope of change over time in the blood flow rate is highly probable to clearly indicate the blood circulation state in the mass of tissue regardless of a type of stimulus causing the change over time in the blood flow rate. In other words, it is also highly probable that a slope when the blood flow is increasing by being warmed from a normal state, a slope when the blood flow is decreasing by being cooled from the normal state, and a slope when the blood flow rate, which has once decreased by being cooled, is again increasing to the normal amount all clearly indicate the blood circulation state.

Yet also, in terms of the change over time in the blood flow associated with a stimulus other than the change in temperature (for example, a friction, an oscillation, or a chemical stimulus or the like), it is also assumed that the slope of change over time in the blood flow is sufficiently probable to clearly indicate the blood circulation state.

Inter alia, it is considered that the slope of the blood flow rate that is measured after the stimulus of warming is applied, the stimulus (warming) is stopped, and then the blood flow undergoes the transient increase particularly well indicates the reallocation of the blood flow increased by the warming. Thus, it is particularly preferable as the index value of the blood circulation state.

It is a newly found fact observed through the above described trial by the inventors of the present invention that the slope of the blood flow rate has the reliability equivalent to the TcPO2 or SPP. Nevertheless, further through the trial by the inventors of the present invention, it is also newly found that a useful action that is not obtainable by the TcPO2 or SPP can be obtained when the slope of the blood flow rate is used as the index value. This useful action is an action to separate a cluster having a worsened blood circulation state, such as the ischemic state, from a cluster not having the worsened blood circulation state.

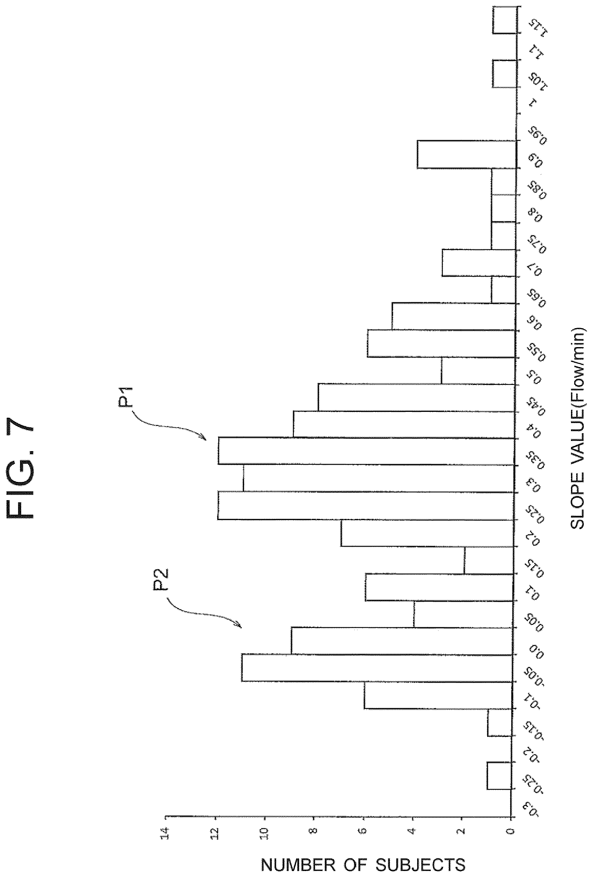

Fig. 7 is a graph illustrating a separation action obtained when the slope of the blood flow rate is used as the index value.

In Fig. 7, the horizontal axis denotes the slope of the blood flow rate and the vertical axis denotes the number of subjects.

When using the slope as the index value, a valley is observed in the distribution of the subjects in the vicinity of the slope of 0.20. It can be observed in the graph that the distribution is separated into two peaks, P1 and P2. The reason why the distribution of the subjects are separated in this way is assumed that it means the phase of the blood circulation state changes with the valley of the distribution serving as a boundary. In order to verify the above assumption, a blood circulation reconstructive operation, of which concrete content differs depending on subjects, was performed with respect to a part of the subjects belonging to the peak P2, which has a slope smaller than 0.20, out of two peaks P1 and P2 (that is, the subjects belonging to the second cluster G2 shown in Fig. 5). As a result, it was confirmed that TcPO2 and SPP significantly increases after the reconstructive operation as compared to before the reconstructive operation. Also, it was confirmed that the slopes of the blood flow rate exceed 0.20 in all cases.

As described above, it was confirmed that the slope of the blood flow rate clearly differs depending on whether the blood circulation state is pathologic or not so as to be a useful as the diagnostic criteria.

It should be noted that it has been conventionally known that this kind of separation never occurs in the distribution of subject when the TcPO2 or SPP is used. Although the diagrammatically illustration will be omitted, it was also confirmed that the separation does not occur in the distribution of the subjects when the TcPO2 or SPP, which is obtained from the same subject cluster through the trial this time, is used.

As described above, it was confirmed that the slope of the blood flow rate is a new index value that is capable of clearly indicating the blood circulation state. According to a method of evaluating the blood circulation state of the present invention using this kind of index value, it makes it possible to accomplish the evaluation in an accurate manner.

It should be noted that, although in the above description the measurement unit, the slope calculation unit and the display unit according to the present invention are exemplarily implemented into a software operating on the personal computer, alternatively the measurement unit, the slope calculation unit or the display unit may be implemented by way of a hardware.

Furthermore, although in the above description a particular example is described in which a device for warming the biological tissue is used as the stimulus applying device according to the invention, alternatively the stimulus applying unit according to the present invention may cool the biological tissue, friction the biological tissue, or apply oscillation or chemical stimulus to the biological tissue.

Yet furthermore, although in the above description a particular example is described in which the blood flow measurement device according to the present embodiment of the present invention is incorporated into the blood flow measurement system with the stimulus applying device, alternatively the blood flow measurement device of the present invention may be separated from the stimulus applying device, or may be a device of another embodiment without being accompanied with the stimulus applying device (for example, an embodiment used for the blood flow measurement of the biological tissue that is, for example, warmed manually by a human hand).

Yet furthermore, although in the above description a particular example is described in which the measurement unit according to the present invention transmits and receives light through the probe affixed to the body of the subject, alternatively the measurement unit according to the present invention may irradiate the subject with light from a position distant from the subject and receives return light at a position distant from the subject.

### REFERENCE SIGNS LIST

10: Blood Flow Measurement System
100: Personal Computer
200: Light Emitting and Light Receiving Unit
300: Probe
400: Warming Sheet
110: Signal Acquisition Unit
120: Flow Rate Calculation Unit
130: Slope Calculation Unit
140: Warming Controller
150: GUI Unit

## Claims

1. A blood flow measurement device comprising:
a measurement unit (110, 120) configured to measure a blood flow rate in capillary vessels of a biological tissue more than once over time;
a slope calculation unit (130) configured to calculate a slope of change over time in the blood flow rate using the blood flow rate of the capillary vessels of the biological tissue measured by the measurement unit and measuring time;
a display unit (150) configured to display a value of the slope as an index value indicating a blood circulation state within a mass of tissue including an underlying portion beneath the capillary vessels of the biological tissue ; and
a stimulus applying device (140) configured to warm the biological tissue, wherein
the measurement unit (110, 120) measures the blood flow rate after the biological tissue being warmed; and
the slope calculation unit (130) calculates the slope that is indicated by decrease in the blood flow rate of the superficial layer of the biological tissue after warming by the stimulus applying device being stopped.

2. The blood flow measurement device according to Claim 1, wherein the measurement unit (110, 120) irradiates the biological tissue with light, receives return light from the biological tissue, and measures the blood flow rate based on a photo-detective signal.

3. The blood flow measurement device according to Claim 2, wherein the measurement unit (110, 120) transmits and receives light to and from the biological tissue by a probe affixed to the biological tissue.

4. The blood flow measurement device according to claim 1, wherein the slope calculation unit (130) calculates the slope with respect to the blood flow rate after a blood flow is temporarily increased due to the biological tissue being warmed.

5. The blood flow measurement device according to any one of preceding claims, wherein the slope calculation unit (130) is configured to calculate a slope of change over time in the blood flow rate with respect to the blood flow rate after the blood flow is temporarily increased due to the biological tissue being warmed using the blood flow rate measured by the measurement unit and measuring time.

6. The blood flow measurement device according to Claim 5, wherein the measurement unit (110, 120) irradiates the biological tissue with light, receives return light from the biological tissue, and measures the blood flow rate based on a photo-detective signal.

7. The blood flow measurement device according to claim 6, wherein the measurement unit (110, 120) transmits and receives light to and from the biological tissue by a probe affixed to the biological tissue.

## Patentansprüche

1. Eine Blutflussmessvorrichtung, die Folgendes umfasst:
eine Messeinheit (110, 120), die ausgebildet ist, um eine Blutflussrate in Kapillargefäßen von biologischem Gewebe mehr als einmal über die Zeit zu messen;
eine Steigungsberechnungseinheit (130), die ausgebildet ist, um eine Steigung der zeitlichen Änderung der Blutflussrate unter Verwendung der von der Messeinheit gemessenen Blutflussrate der Kapillargefäße des biologischen Gewebes und der Messzeit zu berechnen;
eine Anzeigeeinheit (150), die ausgebildet ist, um einen Wert der Steigung als einen Indexwert anzuzeigen, der einen Blutzirkulationszustand innerhalb einer Gewebemasse einschließlich eines darunter liegenden Abschnitts unterhalb der Kapillargefäße des biologischen Gewebes anzeigt; und
die Steigungsberechnungseinheit (130) die Steigung berechnet, die durch die Abnahme der Blutflussrate der oberflächlichen Schicht des biologischen Gewebes angezeigt wird, nachdem die Erwärmung durch die stimulierende Vorrichtung gestoppt wurde.

2. Die Blutflussmessvorrichtung nach Anspruch 1, wobei
die Messeinheit (110, 120) das biologische Gewebe mit Licht bestrahlt, das vom biologischen Gewebe zurückkehrende Licht empfängt und die Blutflussrate auf der Grundlage eines Fotodetektorsignals misst.

3. Die Blutflussmessvorrichtung nach Anspruch 2, wobei
die Messeinheit (110, 120) Licht zu und von dem biologischen Gewebe mittels einer an dem biologischen Gewebe befestigte Sonde sendet und empfängt.

4. Die Blutflussmessvorrichtung nach Anspruch 1, wobei
die Steigungsberechnungseinheit (130) die Steigung in Bezug auf die Blutflussrate berechnet, nachdem ein Blutfluss aufgrund der Erwärmung des biologischen Gewebes vorübergehend erhöht wurde.

5. Die Blutflussmessvorrichtung nach einem der vorhergehenden Ansprüche, wobei
die Steigungsberechnungseinheit (130) ausgebildet ist, um eine Steigung der Änderung über die Zeit in der Blutflussrate in Bezug auf die Blutflussrate zu berechnen, nachdem der Blutfluss aufgrund der Erwärmung des biologischen Gewebes vorübergehend erhöht wird, unter Verwendung der von der Messeinheit gemessenen Blutflussrate und der Messzeit.

6. Die Blutflussmessvorrichtung nach Anspruch 5, wobei
die Messeinheit (110, 120) das biologische Gewebe mit Licht bestrahlt, das vom biologischen Gewebe zurückkehrende Licht empfängt und die Blutflussrate auf der Grundlage eines Fotodetektorsignals misst.

7. Die Blutflussmessvorrichtung nach Anspruch 6, wobei
die Messeinheit (110, 120) Licht zu und von dem biologischen Gewebe mittels einer an dem biologischen Gewebe befestigte Sonde sendet und empfängt.

## Revendications

1. Dispositif de mesure de flux sanguin comprenant :
une unité de mesure (110, 120) configurée pour mesurer le débit sanguin dans des vaisseaux capillaires d'un tissu biologique plusieurs fois au fil du temps ;
une unité de calcul de pente (130) configurée pour calculer la pente du changement dans le temps du débit sanguin en utilisant le débit sanguin des vaisseaux capillaires du tissu biologique mesuré par l'unité de mesure et la période de mesure ;
une unité d'affichage (150) configurée pour afficher la valeur de la pente sous forme de valeur d'indice indiquant l'état du flux sanguin à l'intérieur d'une masse tissulaire englobant une portion sous-jacente sous les vaisseaux capillaires du tissu biologique ; et
un dispositif d'application de stimulus (140) configuré pour réchauffer le tissu biologique,
dans lequel
l'unité de mesure (110, 120) mesure le débit sanguin après que le tissu biologique a été réchauffé ; et
l'unité de calcul de pente (130) calcule la pente qui est indiquée par une diminution du débit sanguin dans la couche superficielle du tissu biologique après que le réchauffement par le dispositif d'application de stimulus a été stoppé.

2. Dispositif de mesure de flux sanguin selon la revendication 1, dans lequel l'unité de mesure (110, 120) irradie le tissu biologique avec une lumière, reçoit la lumière renvoyée par le tissu biologique, et mesure le débit sanguin sur la base d'un signal de photodétection.

3. Dispositif de mesure de flux sanguin selon la revendication 2, dans lequel l'unité de mesure (110, 120) émet et reçoit une lumière vers et depuis le tissu biologique au moyen d'une sonde fixée au tissu biologique.

4. Dispositif de mesure de flux sanguin selon la revendication 1, dans lequel l'unité de calcul de pente (130) calcule la pente en regard du débit sanguin après que le flux sanguin a temporairement augmenté du fait du réchauffage du tissu biologique.

5. Dispositif de mesure de flux sanguin selon l'une quelconque des revendications précédentes, dans lequel l'unité de calcul de pente (130) est configurée pour calculer la pente de changement au fil du temps du débit sanguin par rapport au débit sanguin après que le flux sanguin a temporairement augmenté du fait du réchauffage du tissu biologique au moyen du débit sanguin mesuré par l'unité de mesure et la période de mesure.

6. Dispositif de mesure de flux sanguin selon la revendication 5, dans lequel l'unité de mesure (110, 120) irradie le tissu biologique avec une lumière, reçoit la lumière renvoyée par le tissu biologique, et mesure le débit sanguin sur la base d'un signal de photodétection.

7. Dispositif de mesure de flux sanguin selon la revendication 6, dans lequel l'unité de mesure (110, 120) émet et reçoit une lumière vers et depuis le tissu biologique au moyen d'une sonde fixée au tissu biologique.
